# EUROPEAN PATENT APPLICATION

(11) **EP 3 569 070 A1**
(43) Date of publication of application: **20.11.2019**
(21) Application number: 18739001.8
(22) Date of filing: 11.01.2018
(51) Int. Cl.: A23L 5/00, A23L 33/10, A23L 33/175, A61K 9/48, A61K 31/198, A61K 31/4745, A61K 47/40, A61K 47/44, A61P 3/02, A61P 43/00

(54) **CAPSULE CONTAINING PYRROLOQUINOLINE QUINONE OR SALT THEREOF AND BRANCHED CHAIN AMINO ACID**

(30) Priority: 12.01.2017 JP 2017003627
(71) Applicant: Mitsubishi Gas Chemical Company, Inc., Tokyo 100-8324 (JP)
(72) Inventor: IKEMOTO, Kazuto, Niigata-shi Niigata 950-3112 (JP)
(74) Representative: Müller-Boré & Partner Patentanwälte PartG mbB
(86) International application number: PCT/JP2018/000461
(87) International publication number: WO 2018/131643

(57) **Abstract**

The present invention is a capsule containing pyrroloquinoline quinone or salt thereof, a branched-chain amino acid, and an edible oil or fat or a cyclodextrin as contents of the capsule.

## Description

### Technical Field

The present invention relates to a capsule comprising pyrroloquinoline quinone or a salt thereof and a branched-chain amino acid.

### Background Art

Amino acids are essential nutritional components and thus are frequently supplied as supplements. The antifatigue/healing-related market is expected to continue the growth of recent years, and supplements with highly effective antifatigue performance are desired. A variety of components are used as antifatigue components. Such typical components include caffeine, arginine, taurine, and branched-chain amino acids (also referred to as BCAAs in the following) (refer, for example, to Patent Literature 1). The branched-chain amino acids are known to be antifatigue components (Non-Patent Literature 1).

Disodium pyrroloquinoline quinone has the effect of increasing the mitochondria (Non-Patent Literature 2) which is expected to increase metabolism.

The combination of these two components can be expected, based on antifatigue and mitochondrial activation, to be a dietary supplement ingredient for metabolism and physical exercise.

### Citation List

### Patent Literature

Patent Literature 1: WO 02/034257
Patent Literature 2: Patent Publication JP-A-2012-036094

### Non-Patent Literature

Non-Patent Literature 1: Shiraki et al., 235, Nutrition, Digestion, 59th Annual Meeting of the Japanese Society of Physical Fitness and Sports Medicine
Non-Patent Literature 2: Chowanadisai W, Bauerly KA, Tchaparian E, Wong A, Cortopassi GA, Rucker RB Pyrroloquinoline quinone stimulates mitochondrial biogenesis through cAMP response element-binding protein phosphorylation and increased PGC-1alpha expression. J Biol Chem 285(1), 142-52 (2010)

### Summary

### Technical Problem

BCAAs exhibit an increase in odor, for example, upon contact with moisture or when heated. In the case of combination with disodium pyrroloquinoline quinone, it has been found that BCAAs also give off odor when mixed in powder form and that, in particular in the case of contact with moisture, their decomposition is promoted and thus the generation of odor is also promoted. Countermeasures here have therefore been required.

For consumption by dietary intake, pyrroloquinoline quinone is frequently provided as a tablet or capsule, and soft capsules containing pyrroloquinoline quinone are known (Patent Literature 2). However, no cases are unknown in which pyrroloquinoline quinone is mixed with branched-chain amino acid. In addition, the odor of branched-chain amino acids is not suppressed by ordinary tablet production.

The present invention addresses the problem of suppressing odor generation and thereby enabling the joint intake of branched-chain amino acid with pyrroloquinoline quinone or a salt thereof.

### Solution to Problem

As a result of extensive and intensive investigations in order to solve the aforementioned problem, the present inventors achieved the present invention through the following means.
[1] A capsule comprising, as contents of the capsule: pyrroloquinoline quinone or salt thereof;
   a branched-chain amino acid; and
   an edible oil or fat or a cyclodextrin.
[2] The capsule according to [1], characterized in that the salt of pyrroloquinoline quinone is disodium pyrroloquinoline quinone.
[3] The capsule according to [2], characterized in that the disodium pyrroloquinoline quinone is an anhydrous crystal.
[4] The capsule according to any of [1] to [3], wherein the pyrroloquinoline quinone or salt thereof, branched-chain amino acid, and edible oil or fat are combined in the following ratio: pyrroloquinoline quinone or salt thereof : branched-chain amino acid : edible oil or fat = 1 : 0.2 to 1200 : 0.1 to 1000.
[5] A method for producing the capsule according to any of [1] to [4], comprising filling the capsule with the contents of the capsule after being subject to a drying treatment.
[6] A method of storing the capsule according to any of [1] to [4] under 10% or less of oxygen.
[7] A method for suppressing an odor of a branched-chain amino acid, comprising a step of bringing pyrroloquinoline quinone or salt thereof, a branched-chain amino acid, and an edible oil or fat or a cyclodextrin into contact with each other within a capsule.

### Advantageous Effects of Invention

Pyrroloquinoline quinone promotes the decomposition of branched-chain amino acids under contact with moisture and thus promotes the generation of the odor that originates with branched-chain amino acids. According to the present invention, it is possible to suppress the generation of the odor that originates with branched-chain amino acids and thereby facilitate the joint intake of branched-chain amino acids with pyrroloquinoline quinone or a salt thereof. This makes it possible to obtain effective and odor-inhibited functional food products.

### Brief Description of Drawings

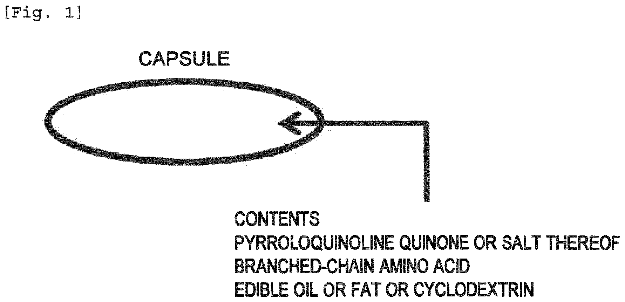

Fig. 1 shows a capsule structure and the contents filled into the capsule.

### Description of Embodiments

Embodiments of the present invention are described in the following.

The present invention is a capsule that contains pyrroloquinoline quinone or a salt thereof, branched-chain amino acid, and an edible oil or fat.

The pyrroloquinoline quinone used in the present invention is a substance with the structure given in formula 1. The present invention may also use a salt of pyrroloquinoline quinone.

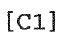

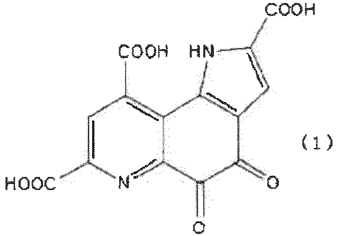

The pyrroloquinoline quinone or salt thereof used in the present invention is available as a commercial product or may be produced by a known method. The salts of pyrroloquinoline quinone can be exemplified by the alkali metal salts, alkaline-earth metal salts, and ammonium salts of pyrroloquinoline quinone. The alkali metal salts are preferred.

The alkali metals salts of pyrroloquinoline quinone used in the present invention can be exemplified by the sodium, potassium, lithium, cesium, and rubidium salts. The sodium salts and potassium salts are more preferred as the alkali metal from the standpoint of ease of acquisition. The alkali metal salt of pyrroloquinoline quinone may be an alkali metal salt substituted by 1 to 3 alkali metals, and the substituents may be any of the following: the mono-alkali metal salt, di-alkali metal salt, or tri-alkali metal salt. The di-alkali metal salts are preferred. The disodium salt and dipotassium salt are particularly preferred.

Disodium pyrroloquinoline quinone is generally commercially available as a hydrated crystal. This hydrated crystal is commonly the trihydrate and contains at least 10% water. However, when a hydrated crystal is used by the present invention, it is preferred to bring down its water content to 8% or less by drying. The anhydrous crystal is particularly preferred. Removal of the water from the disodium pyrroloquinoline quinone can be carried out by drying by an ordinary method.

The amount of pyrroloquinoline quinone or salt thereof contained in the capsule according to the present invention may be adjusted as appropriate in conformity with the use for the final product and the magnitude of the odor to be resolved. While not intended as a limitation, the pyrroloquinoline quinone or salt thereof may be 1 to 40 mg in the case of incorporation as a supplement.

Branched-chain amino acids (BCAA) collectively refer to valine, leucine, and isoleucine, which are included in the nine essential amino acids that cannot be synthesized in the human body, and are important nutrients that act as an energy source in muscle. The branched-chain amino acids are present in large quantities in, for example, meat, fish, dairy products, and eggs. However, with foods, due to ingestion as protein, several hours are required for degradation to the branched-chain amino acids and absorption. Thus, the ingestion of branched-chain amino acids in the form of a beverage or supplement is effective for obtaining an efficient stress recovery effect with branched-chain amino acids when stress actually occurs. An antifatigue effect can be obtained in humans by the intake of 20 to 1200 mg per one dose for the amount of branched-chain amino acid ingestion. The branched-chain amino acid is preferably isoleucine. 20 to 1200 mg branched-chain amino acid is preferably contained in the capsule.

The method for producing the branched-chain amino acid is not particularly limited, and production can be carried out by, for example, fermentation, synthesis, or purification. Commercial products may be used. Common branched-chain amino acids can be used for the branched-chain amino acids used in the present invention; however, pure products produced by fermentation are preferred from the standpoints of taste and management of component concentration.

Types of the edible oil or fat are not particularly limited as long as they are ingestible by humans and/or nonhuman mammals. It can be exemplified by plant oils and fats such as olive oil, sesame oil, rice oil, safflower oil, soybean oil, corn oil, rapeseed oil, palm oil, palm olein, palm kernel oil, sunflower oil, grape oil, cottonseed oil, coconut oil, and peanut oil, and by edible oils and fats such as neutral fatty acid triglycerides, squalene, fish oil, deeply hydrogenated oils, cocoa butter, butter, beeswax, beef tallow, and lard. A solid or liquid may be used for the edible oil or fat. In the case of a solid, an interim melting and mixing to uniformity is desirably carried out. Preferably 10 to 1000 mg edible oil or fat is contained in the capsule.

The odor in the present invention refers to an odor such as a metallic odor or a raw or fishy odor such as an aldehyde odor. Thus, "odor suppression" refers to a condition wherein off-odors, such as a metallic odor or a raw or fishy odor, are suppressed to such a degree that at the time of ingestion the consumer is unconcerned.

Odor suppression may also be achieved in the present invention by the addition of cyclodextrin, either in place of the edible oil or fat or in combination therewith. An alpha-, beta-, or gamma-cyclodextrin can be used as the cyclodextrin.

From the standpoint of suppressing the odor of BCAAs, there are no particular limitations on how the capsule is taken or on the amount of intake of each component. However, in order to obtain the expected effect of pyrroloquinoline quinone or salt thereof, such as improved brain function or skin moisturization, the amount of intake of pyrroloquinoline quinone or salt thereof is, per one dose, 1 to 100 mg, preferably 2 to 50 mg, and more preferably 5 to 30 mg.

The amount of the individual components incorporated in the capsule varies with, for example, the extent of the odor, the type of the desired product, and so forth. However, the pyrroloquinoline quinone or salt thereof is preferably contained at 1 to 100 mg, the branched-chain amino acid is preferably contained at 20 to 1200 mg, and the edible oil or fat is preferably contained at 10 to 1000 mg. The capsule may more preferably contain 2 to 50 mg and still more preferably 5 to 30 mg pyrroloquinoline quinone or salt thereof, more preferably 100 to 1200 mg branched-chain amino acid, and more preferably 100 to 1000 mg edible oil or fat. In accordance with a preferred embodiment, the weight ratio among the contents is pyrroloquinoline quinone or salt thereof : branched-chain amino acid : edible oil or fat = 1 : 0.2 to 1200 : 0.1 to 1000. With regard to the ease of odor generation, larger amounts of branched-chain amino acid facilitate odor emission; for pyrroloquinoline quinone or salt thereof, the dependency on amount is low since it is catalytic. Use of the present invention makes possible a free configuration in accordance with the range given above.

In the case of producing hard capsules according to the production method of the present invention, it can be carried out by filling pyrroloquinoline quinone or salt thereof and edible oil or fat or cyclodextrin into the hard capsule and closing the capsule.

In the case of producing soft capsules, it can be carried out by dispersing the pyrroloquinoline quinone or salt thereof in the edible oil or fat, and, after these have been brought into fluid state, enclosing them with a gelatin sheet and taking off in soft capsule form using a die. Low humidity is a preferred condition for soft capsule fabrication. For example, 0 environmental humidity brought about using anhydrous calcium chloride is preferred.

An example of the capsule structure is given in Fig. 1. As shown in Fig. 1, pyrroloquinoline quinone or salt thereof, BCAA, and edible oil or fat or cyclodextrin are introduced and present within the capsule. Odor can be suppressed by the capsule and by the edible oil or fat or cyclodextrin used as an additive. While not intending to be bound by theory, it is thought that the odor suppression is achieved through an inhibition of the BCAA decomposition reactions, through odor adsorption, and through an inhibition by the capsule of moisture adsorption. The capsule used may be either a hard capsule or a soft capsule. The material may be gelatin or a cellulosic material. A material having a low moisture permeability is more preferred.

Drying the contents to be filled into the capsule is effective for suppressing odor generation. The odor can be suppressed by a drying in which the branched-chain amino acid and pyrroloquinoline quinone mixture to be filled into the capsule is treated under conditions of a low humidity of not more than 10%. The odor can also be suppressed by drying with the application of heat and drying under the application of reduced pressure. Drying under reduced pressure is a more preferred drying condition. Dry air may be flowed into the capsule during capsule filling in order to reduce the humidity, and the moisture contained in the powder may be suppressed by bringing the mixture into a dry state. Odor generation is more effectively suppressed by bringing the moisture contained in the starting materials for the components contained in the capsule to 5% or less.

The contents can be filled into the capsule after being subject to the drying treatment. The contents are preferably subject to a drying treatment prior to mixing.

The drying time can be varied as necessary considering, for example, the amount of moisture present in the contents. The drying time is, for example, 0.1 second to 3 hours.

A manual or automatic method can be used for capsule filling. More rapid processing is also preferred from the standpoint of production costs.

The present invention may be a drug composition; a quasi-drug composition; and a so-called health food, a dietary supplement, a nutraceutical food, a food for specified health use, a functional food, and an animal feed which have a pharmacological effect.

When the capsule according to the present invention is used for, for example, a drug composition, a quasi-drug composition, or a food composition such as a so-called health food having a pharmacological effect, a dietary supplement, and so forth, ingestion may be made even easier by the addition as appropriate of a compounding agent, e.g., a binder, within a range in which the odor-suppression effect is not impaired.

More particularly, an excipient or vehicle, and as necessary a binder, disintegrant, lubricant, colorant, flavoring agent, odor-masking agent, and so forth can be added to the composition in the present invention, and tablets, coated tablets, granules, powders, capsules, and the like can be produced by a conventional method.

The additives commonly used in the pertinent field may be used as these additives. The excipient or vehicle can be exemplified by lactose, sucrose, sodium chloride, glucose, starch, calcium carbonate, kaolin, microcrystalline cellulose, and silicic acid. The binder can be exemplified by water, ethanol, propanol, simple syrup, glucose, starch, gelatin, carboxymethyl cellulose, hydroxypropyl cellulose, hydroxypropyl starch, methyl cellulose, ethyl cellulose, calcium phosphate, and polyvinylpyrrolidone. The disintegrant can be exemplified by dry starch, sodium alginate, agar powder, sodium bicarbonate, calcium carbonate, sodium lauryl sulfate, glycerol monostearate, and lactose. The lubricant can be exemplified by purified talc, stearate salts, borax, and polyethylene glycol, while the flavoring agent can be exemplified by sucrose, orange peel, citric acid, and tartaric acid.

Storage under low oxygen of not more than 10% and storage in an environment lacking oxygen are effective for achieving a greater odor suppression. Thus, the capsule is preferably stored with an oxygen absorber such as AGELESS (registered trademark).

In addition, the following, for example, may be added as appropriate during capsule production within a range in which the odor-suppression effect is not impaired: other food ingredients, i.e., various sugars, as well as emulsifiers, thickeners, sweeteners other than cyclodextrin, acidulants, flavors, amino acids, and fruit juices. Specifically, the following, for example, may be added: saccharides such as sucrose, isomerized sugars, glucose, fructose, palatinose, trehalose, lactose, and xylose; sugar alcohols such as sorbitol, xylitol, erythritol, lactitol, palatinitol, reduced starch syrup, and reduced maltose syrup; high-intensity sweeteners such as aspartame, stevia, acesulfame potassium, and sucralose; emulsifiers such as sucrose/fatty acid ester, glycerol/fatty acid ester, and lecithin; thickeners, (stabilizers) such as carrageenan, xanthan gum, guar gum, pectin, and locust bean gum; acidulants such as citric acid, lactic acid, and malic acid; and juices such as lemon juice, orange juice, and berry juices. In addition to the preceding, vitamins such as vitamin A, the vitamin Bs, vitamin C, vitamin D, and vitamin E and minerals such as calcium, iron, manganese, and zinc can be added.

The capsule according to the present invention is preferably stored at low humidity. A desiccant-containing bottle or individual packaging of each capsule is preferred in order to prevent the entry of moisture.

### Examples

The present invention is more specifically described through the examples provided below, but the present invention is not limited to or by these examples.

Unless specifically indicated otherwise, the following were used in these examples: 100-mL mayonnaise bottles and, as the disodium pyrroloquinoline quinone, BioPQQ (registered trademark) (9% to 12% water content) from Mitsubishi Gas Chemical Company, Inc.

### Examples 1 to 3

The capsule used was a hydroxypropyl methyl cellulose #0 capsule (0.68 mL dose) having a structure separable in two (sold by Great Island Co. Ltd.).

The capsule was divided in two and to one side was added the particular odor-suppressing additive shown in Table 1 in addition to a preliminary prepared mixture of 10 mg disodium pyrroloquinoline quinone and 50 mg leucine. After standing for 1 hour under low humidity (environmental humidity of 0 achieved with anhydrous calcium chloride), the capsule was closed and finished. The capsule was placed in a mayonnaise bottle brought to a humidity of 70% and was stored at 30°C. The odor in the capsule was tested by smell after one day and after five days.

**[Table 1]**

| example | additive | odor after one day |
|---|---|---|
| 1 | medium-chain fatty acid oil or fat (ODO, The Nisshin Oillio Group, Ltd.), 60 mg | none |
| 2 | beta-cyclodextrin (60 mg) | none |
| 3 | gamma-cyclodextrin (60 mg) | none |

These results demonstrated that the odor could be suppressed for at least one day for the working time until insertion in an ordinary bottle or pouch. The medium-chain fatty acid oil or fat, an edible oil or fat, suppressed odor better than the cyclodextrin.

### Comparative Examples: Elucidation Using Accelerated Testing of Cause of Odor for Mixtures

BioPQQ (registered trademark) from Mitsubishi Gas Chemical Company, Inc. was used for the disodium pyrroloquinoline quinone.

The compositions indicated below were placed in 100-mL mayonnaise bottles, and were placed in mayonnaise bottles brought to a humidity of 70% and stored at 30°C. The odor in the bottle was tested by smell after one day.

**[Table 2]**

| comparative example | | odor after one day |
|---|---|---|
| 1 | 10 mg disodium pyrroloquinoline quinone and 50 mg leucine | present |
| 2 | 10 mg disodium pyrroloquinoline quinone and 50 mg leucine, 1 mL water | very strong |

This experiment demonstrates that odor is generated by the combination of branched-chain amino acid and disodium pyrroloquinoline quinone. According to results not shown, odor is also emitted when leucine alone is brought into contact with water. As shown here, odor is readily emitted when storage is carried out without filling into a capsule.

### Examples 4 to 6: Experiments with Different Edible Oils and Fats

The capsule used was a hydroxypropyl methyl cellulose #0 capsule (0.68 mL dose) (sold by Great Island Co. Ltd.).

The moisture content was brought to 5% or less by drying under reduced pressure for at least 1 hour at 120°C.

10 mg disodium pyrroloquinoline quinone and 60 mg of the particular edible oil or fat were dried in a vacuum dryer for 1 hour at 120°C. The dried material was mixed with 50 mg leucine and this was filled into the capsule and finishing was carried out. The capsule was placed in a mayonnaise jar that had been brought to a humidity of 70% and was stored at 30°C. The odor in the capsule was tested by smell after one day.

**[Table 3]**

| example | edible oil or fat | odor after one day |
|---|---|---|
| 4 | medium-chain fatty acid oil or fat (ODO, The Nisshin Oillio Group, Ltd.), 60 mg | none |
| 5 | deeply hydrogenated oil (TP-9, NOF Corporation), 60 mg | none |
| 6 | cocoa butter (Epron), 60 mg | none |

The odor could be suppressed by using a dried disodium pyrroloquinoline quinone and mixing with an edible oil or fat.

### Comparative Example 3: Tablet 1

10 mg disodium pyrroloquinoline quinone, 50 mg leucine, and 140 mg lactose were made into a tablet with a diameter of 5 mm using a tablet press. A strong odor was present when this was placed in a mayonnaise bottle brought to a humidity of 70% and the odor was tested by smell after the elapse of one day at 30°C.

### Comparative Example 4: Tablet 2

10 mg disodium pyrroloquinoline quinone, 50 mg leucine, and 140 mg rice flour were made into a tablet with a diameter of 5 mm using a tablet press. Odor was present when this was placed in a mayonnaise bottle brought to a humidity of 70% and the odor was tested by smell after the elapse of one day at 30°C. Odor suppression could not be achieved when the dosage form was a tablet rather than a capsule.

### Example 7

10 mg of disodium pyrroloquinoline quinone and 60 mg of a medium-chain fatty acid oil or fat (ODO, The Nisshin Oillio Group, Ltd.) were placed in a vacuum dryer and were dried for 1 hour at 120°C. The dried material was mixed with 40 mg leucine, 5 mg valine, and 5 mg isoleucine and this was filled into a capsule and finishing was carried out. Odor did not occur when the capsule was placed in a mayonnaise bottle brought to a humidity of 70% and the odor was tested by smell after the elapse of one day at 30°C. The present invention was thus also effective with a plurality of branched-chain amino acids.

### Comparative Example 5

10 mg disodium pyrroloquinoline quinone was mixed with 40 mg leucine, 5 mg valine, and 5 mg isoleucine. The mixture was placed in a mayonnaise bottle brought to a humidity of 70% and was stored at 30°C. Odor generation had occurred when the odor was tested by smell after one day.

### Industrial Applicability

The present invention is effective for sectors such as food products in general, functional foods, pet foods, the drug sector, and so forth.

## Claims

1. A capsule comprising, as contents of the capsule:
pyrroloquinoline quinone or salt thereof;
a branched-chain amino acid; and
an edible oil or fat, or a cyclodextrin.

2. The capsule according to claim 1, **characterized in that** the salt of pyrroloquinoline quinone is disodium pyrroloquinoline quinone.

3. The capsule according to claim 2, **characterized in that** the disodium pyrroloquinoline quinone is an anhydrous crystal.

4. The capsule according to any one of claims 1 to 3, wherein the pyrroloquinoline quinone or salt thereof, branched-chain amino acid, and edible oil or fat are combined in the following ratio: pyrroloquinoline quinone or salt thereof : branched-chain amino acid : edible oil or fat = 1 : 0.2 to 1200 : 0.1 to 1000.

5. A method for producing the capsule according to any one of claims 1 to 4, comprising filling the capsule with the contents of the capsule after being subject to a drying treatment.

6. A method of storing the capsule according to any one of claims 1 to 4 under 10% or less of oxygen.

7. A method for suppressing an odor of a branched-chain amino acid, comprising a step of bringing pyrroloquinoline quinone or salt thereof, a branched-chain amino acid, and an edible oil or fat or a cyclodextrin into contact with each other within a capsule.
